# EUROPEAN PATENT APPLICATION

(11) **EP 2 005 965 A1**
(43) Date of publication of application: **24.12.2008**
(21) Application number: 07737337.1
(22) Date of filing: 16.02.2007
(51) Int. Cl.: A61K 35/02, A61P 35/00, C02F 1/68

(54) **WATER HAVING ANTICANCER ACTION AND METHOD FOR PRODUCING THE SAME**

(30) Priority: 20.03.2006 JP 2006076790; 26.01.2007 JP 2007015753
(71) Applicant: Fukai, Toshiharu, Nagano, 3860002 (JP)
(72) Inventor: Fukai, Toshiharu, Nagano, 3860002 (JP)
(74) Representative: Betten & Resch
(86) International application number: PCT/JP2007/053348
(87) International publication number: WO 2007/108275

(57) **Abstract**

[SUMMARY]

[OBJECT] To provide water that is able to suppress proliferation of cancer cells by taking it in the human body through ingestion or injection thereof and is free of side effects and a method for making same.

[SOLVING MEANS] Soft water generators 10, 12, each having an ion exchange resin 32 therein, an ion generator having tourmaline therein, and a rock accommodating container having a rock 54 selected from igneous rocks and containing a large amount of silicon dioxide are connected in series in this order, and water is passed through the ion exchange resin 32, the tourmaline 46 and the rock 54 selected from igneous rocks and containing a large amount of silicon dioxide in this order. The resulting water contains large amounts of a hydrogen ion (H⁺) and a hydronium ion (H₃O⁺). When this water is ingested as ordinary drinking water or injected in the human body along with the ingestion, cancer cells can be suppressed from division and proliferation.

## Description

### [TECHNICAL FIELD]

This invention relates to water having anticancer activity of suppressing proliferation of cancer cells in vivo when taken in the human body by ingestion as a drink or by injection and also to a method for making such water.

### [BACKGROUND ART]

Hitherto, studies for treating cancer patients have been made by many doctors and oncologists. For a measure of treating cancers by in vivo ingestion, mention is made, mainly, of medicines. Up to now, many inventions relating to anticancer drugs have been made and, for example, Patent Literature 1 indicated below proposes such drugs.

Aside from drugs, those known in the art for treating cancers by in vivo ingestion include fungi or mushrooms such as agaricus mushroom, meshimakobu (phellinus linteus) and the like. According to popular belief, it has been said that agaricus mushrooms or meshimakobu may be decocted and the resulting decoction is drunk, or may be directly ingested as a food, with which cancer cells are suppressed from division and proliferation. In this connection, however, the Ministry of Health, Labor and Welfare of Japan has not recognized the anticancer activity of such mushrooms as agaricus mushrooms, meshimakobu and the like.

### [Patent Literature 1]

Japanese Laid-Open Patent Application No. 2004-352673

### [DISCLOSURE OF THE INVENTION]

### [Problem to be Solved by the Invention]

The development of anticancer drugs generally takes much labor and cost and thus, difficulties are involved in such drugs being developed by those other than big pharmaceutical companies. Moreover, when drugs having anticancer activity are administered, side effects such as of hair loss may develop. In general, if two or more drugs are used in combination, there is concern that unexpected side effects may occur. For patients having other disease in combination, two or more drugs cannot be simultaneously used in relief unless it has been confirmed that there is no side effect of each other's drugs. Besides, whether mushrooms such as agaricus, meshimakobu and the like provided as a drink or food have, in fact, anticancer activity has never been scientifically proven yet.

The invention has been made under these circumstances in the art and has for its object the provision of water that is capable of suppressing cancer cells in vivo from division and proliferation by ingestion for a drink or by injection and is free of side effects and also of a method for making such water.

### [Means for Solving the Problem]

A method for making water having anticancer activity (Example 1) according to the invention is characterized by passing water through an ion exchange resin, tourmaline and a rock selected from igneous rocks and containing a large amount of silicon dioxide in this order. A method for making water having anticancer activity (Example 2) according to the invention is characterized by passing water through an ion exchange resin, a rock selected from igneous rocks and containing a large amount of silicon dioxide, and tourmaline in this order. A method for making water having anticancer activity (Examples 3 and 4) according to the invention is characterized by passing water firstly through either of tourmaline or a rock selected from igneous rocks and containing a large amount of silicon dioxide, and secondly through the other. The invention is characterized in that a metal that gives no adverse influence on the human body by dissolution in water is mixed with the tourmaline. The invention is characterized in that the rock selected from igneous rocks and containing a large amount of silicon dioxide consists of at least one member selected from rhyolites including obsidian, pearlite and pitchstone and granites. The invention is characterized in that the metal consists of at least one of aluminium, a stainless steel and silver. The invention is characterized in that a ratio by weight between the tourmaline and the metal is at 10:1 to 1:10. The invention is characterized in that the tourmaline is one obtained by mixing with a ceramic material at a ratio thereof by weight of not smaller than 10% and heating the mixture at 800°C or over. The invention is characterized in that the rock selected from igneous rocks and containing a large amount of silicon dioxide is one obtained by heating at 800°C or over. The invention is characterized in that the ion exchange resin generates a sodium ion by ion exchange.

Water having anticancer activity (Example 1) according to the invention is characterized by passing water through an ion exchange resin, tourmaline and a rock selected from igneous rocks and containing a large amount of silicon dioxide in this order. Water having anticancer activity (Example 2) according to the invention is characterized by passing water through an ion exchange resin, a rock selected from igneous rocks and containing a large amount of silicon dioxide, and tourmaline in this order. Water having anticancer activity (Examples 3 and 4) according to the invention is characterized by passing firstly through either of tourmaline or a rock selected from igneous rocks and containing a large amount of silicon dioxide, and secondly through the other. The invention is characterized in that a metal that gives no adverse influence on the human body by dissolution in water is mixed with the tourmaline. The invention is characterized in that the rock selected from igneous rocks and containing a large amount of silicon dioxide consists of at least one member selected from rhyolites including obsidian, pearlite and pitchstone and granites. The invention is characterized in that the metal consists of at least one of aluminium, a stainless steel and silver. The invention is characterized in that a ratio by weight between the tourmaline and the metal is at 10:1 to 1:10. The invention is characterized in that the tourmaline is one obtained by mixing with a ceramic material at a ratio thereof by weight of not smaller than 10% and heating the mixture at 800°C or over. The invention is characterized in that the rock selected from igneous rocks and containing a large amount of silicon dioxide is one obtained by heating at 800°C or over. The invention is characterized in that the ion exchange resin generates a sodium ion by ion exchange.

### [Effects of the Invention]

The invention relates to the preparation of a specific type of water (hereinafter referred to as "artificially created water") by passing water through three types of materials including an ion exchange resin, tourmaline, and a rock selected from igneous rocks and containing a large amount of silicon dioxide, or passing water through two types of materials including tourmaline and a rock selected from igneous rocks and containing a large amount of silicon dioxide. When the artificially created water is ingested by drinking or injected, cancer cells occurring in the human body are suppressed from division and proliferation. In the practice of the invention, since artificially created water is only ingested as daily drinking water, patients having other disease in combination can safely administer a drug for other disease. Since the artificially created water is suited for drinking, there is no concern that side effects such as of hair loss develop as would otherwise occur when administering an anticancer drug.

### [BEST MODE FOR CARRYING OUT THE INVENTION]

This invention provides a specific type of water adapted for suppressing cancer cells occurring in the human body from division and proliferation by taking it in the human body by drinking or injection and also a method for making the water.

### [Example 1]

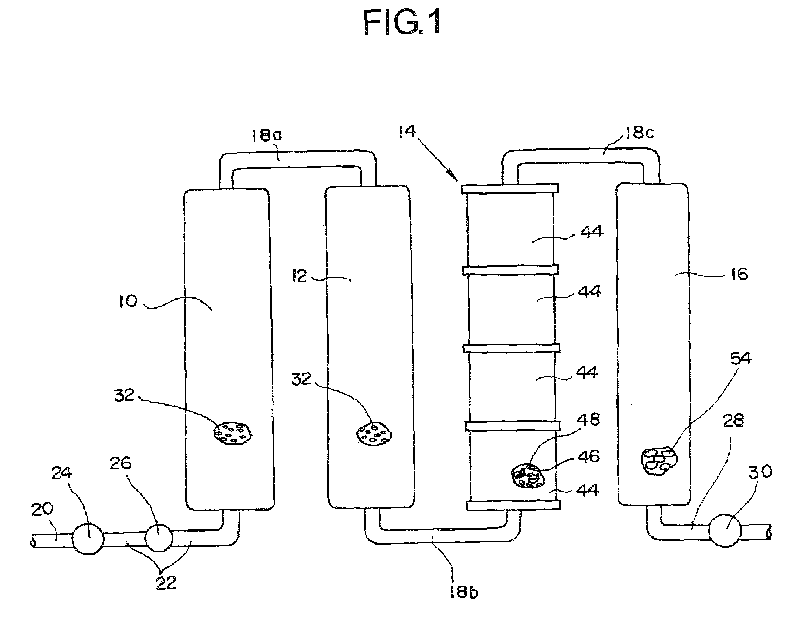

Examples of the invention are illustrated with reference to the accompanying drawings. Fig. 1 is a schematic view of an arrangement showing an example of an apparatus of making water having anticancer activity according to the invention. A first soft water generator 10, a second soft water generator 12, an ion generator 14, and a rock accommodating container 16 are, respectively, connected in series through connection pipes 18a, 18b and 18c. Pressurized water such as, for example, city water is supplied to the first soft water generator 10 from a water supply pipe 20 through a connection pipe 22. An on-off inlet valve 24 such as a bib cock is provided between the water supply pipe 20 and the connection pipe 22 with a check valve 26 being provided on the way of the connection pipe 22. A delivery pipe 28 is provided at the outlet side of the rock accommodating container 16 and has an outlet on-off valve 30 at the tip or on the way of the delivery pipe 28.

With city water, water fed from the water supply pipe 20 is passed to the first soft water generator 10, second soft water generator 12, ion generator 14 and rock accommodating container 16 in this order and is collected from the delivery pipe 28 by opening the outlet on-off valve 30. With waters other than city water, although not shown, water in a water reservoir is introduced into the first soft water generator 10 via the water supply pipe 20 by means of a pump. In this case, the check valve 26 is provided between the pump and the first soft water generator 10.

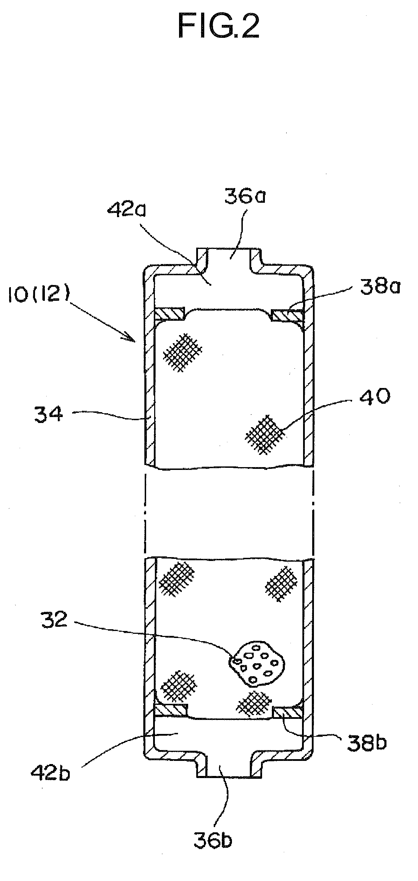

The first soft water generator 10 and the second soft water generator 12, respectively, accommodate therein a particulate ion exchange resin 32 in large amounts, with its section being shown in Fig. 2. The soft water generators 10, 12, respectively, have a body 34 which is cylindrical in shape and has water outlet and inlet ports 36a, 36b at upper and lower end faces thereof, respectively. The cylindrical body 34 is provided with shield members 38a, 38b at inner walls kept slightly away from the upper and lower end faces as having an opening at the center thereof, respectively. The ion exchange resin 32 contained in a fine net 40 is accommodated between the paired shield members 38a, 38b. The reason why the shield members 38 each having the opening at the center thereof are provided at the inner walls positioned slightly away from the upper and lower outlet and inlet ports 36a, 36b is that the net 40 containing the ion exchange resin 32 is disposed between the paired shield members 38 to establish spaces 42a, 42b in the vicinity of the outlet and inlet ports 36a, 36b, respectively. The reason why water is permitted to be charged and discharged from the central openings of the shield members 38a, 38b is that water is invariably brought into contact with the ion exchange resin 32. In addition, the reason why the ion exchange resin 32 is placed in the net 40 is that the particulate ion exchange resin 32 can be wholly remove along with the net 40 upon removal of the particulate ion exchange resin 32 for washing.

The first and second soft water generators 10, 12 , respectively, have a height of 80 cm and an inner diameter of 10 cm, for example. The height of accommodation of the ion exchange resin 32 is set, for example, at 70 cm (permitting the upper and lower spaces 42a, 42b to be established). The height of accommodation of the ion exchange resin 32 should be sufficient to satisfactorily effect ion exchange. On the other hand, when the height of the accommodated ion exchange resin 32 becomes too great (e.g. when the height of the accommodated ion exchange resin 32 is over about 200 cm), the ion exchange resin 32 becomes resistant to the passage of water, resulting in a reduced flow rate of water passing through the inside of the soft water generator. Accordingly, the height of accommodation of the ion exchange resin 32 should be determined as not reducing the flow rate. Two containers are used for accommodation of the ion exchange resin 32, for which the first and second soft water generators 10, 12 are suppressed in height to substantially such a level as the ion generator 14 and the rock accommodating container 16 and the flow rate is avoided from being reduced owing to the pressure loss of water passing therethrough. The two soft water generators 10,12 may be combined together to provide one soft water generator. Depending on the flow rate of water, the inner diameter of the soft water generators, the height of the accommodated ion exchange resin 32 and the number of the soft water generators connected in series may be optionally determined.

The ion exchange resin 32 serves to eliminate metal ions such as Ca²⁺, Mg²⁺, Fe²⁺ and the like from water to provide soft water and especially, to lower the hardness of water to a level near to zero. The ion exchange resin 32 used is, for example, a strongly acidic cationic exchange resin (R₂SO₃Na) obtained by uniform sulfonation of a sphere-shaped styrene-divinylbenzene copolymer. The ion exchange resin 32 undergoes the following ion exchange reactions with the metal ions such as Ca²⁺, Mg²⁺, Fe²⁺ and the like.

2RzSO₃Na + Ca²⁺ → (RzSO₃)₂Ca + 2Na⁺

2RzSO₃Na + Mg²⁺ → (RzSO₃)₂Mg + 2Na⁺

2RzSO₃Na + Fe²⁺ → (RzSO₃)₂Fe + 2Na⁺

More particularly, the passage through the ion exchange resin 32 permits Ca²⁺, Mg²⁺, Fe²⁺ and the like to be eliminated from water. The use of the strongly acidic cationic exchange resin (RzSO₃Na) as the ion exchange resin 32 results in the formation of a sodium ion (Na⁺). The ion exchange resin 32 may be one which is able to produce ions other than Na⁺ and should preferably one which generates Na⁺. If city water is used, chlorine is also contained therein aside from the metal ions such as Ca²⁺, Mg²⁺, Fe²⁺ and the like. No change occurs on chlorine when city water is passed through the ion exchange resin 32.

On the other hand, when water (H₂O) is passed through the ion exchange resin 32, the following changes take place.

H₂O → H⁺ + OH- (1)

H₂O + H⁺ → H₃O⁺ (2)

As shown in (1) and (2) above, the passage through the ion exchange resin 32 leads to the generation of a hydroxide ion (OH-) and a hydronium ion (H₃O⁺).

In this way, where water used is hard water, the passage through the ion exchange resin 32 permits metal ions such as Ca²⁺, Mg²⁺, Fe²⁺ and the like to be eliminated from water, thereby providing soft water. The passage through the ion exchange resin 32 also results in the generation of Na⁺, OH- and a hydronium ion (H₃O⁺) in water. In this connection, however, chlorine (Cl) contained in city water is not ionized and is passed as it is. It will be noted that no Na⁺ may be formed depending on the type of ion exchange resin 32.

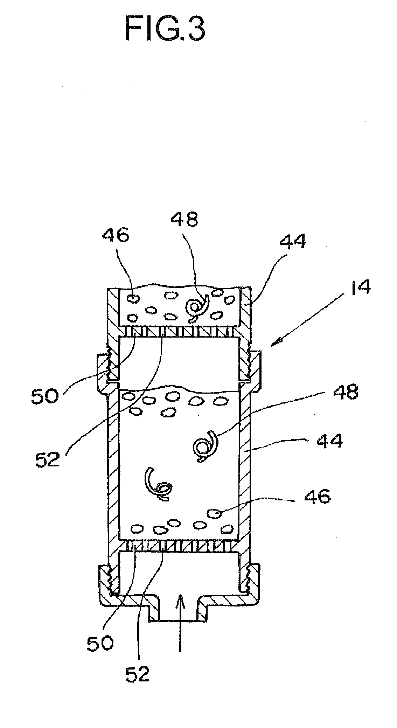

Next, a partial section of the ion generator 14 is shown in Fig. 3. The ion generator 14 includes a plurality of cartridges 44 vertically located at the same position and connected in series. Individual cartridges 44 have either particulate tourmaline 46 alone or a mixture of the particulate tourmaline 46 and a plate-shaped metal 48 accommodated therein. Tourmaline has plus and minus electrodes, with which water is allowed to have an electromagnetic wave having a wavelength of 4 to 14 micrometers, and clusters of water are cleaved off, thereby generating a hydronium ion (H₃O⁺). The energy of the electromagnetic wave having a wavelength of 4 to 14 micrometers is at 0.004 watts/cm². The tourmaline 46 used herein may consist of fine pieces of tourmaline, or may be a tourmaline mixture called tourmaline pellets, which are commercially available as containing tourmaline, a ceramic material and aluminium oxide (which may contain silver therein in some case) at mixing ratios by weight of about 10:80:10. The ceramic material contained in the tourmaline pellets acts to separate the plus and minus electrodes from each other. The tourmaline 46 may be made by mixing not less than 10 wt% of tourmaline with a ceramic material and heating at 800°C or over, by which the tourmaline 46 that disappears within a given period of time (e.g. within about 3 months for a pellet diameter of 4 mm) under agitation in water can be made. Water is passed through the ion exchange resin 32 to provide soft water whose hardness is close to zero, in which the particles of the tourmaline 46 are frictionally contacted with one another. With the soft water whose hardness is close to zero, deposition of magnesium and calcium on the minus electrode of the tourmaline 46 is inhibited, thereby preventing the role of the tourmaline 46 as plus and minus electrodes from lowering.

The metal 48 used is at least one of aluminium, a stainless steel and silver. The metal 48 should preferably be one which does not undergo corrosion in water and is insoluble in water and which does not adversely influence the human body. Of these metals 48, aluminium has the bactericidal or antifungal action and the bleaching function, and a stainless steel has the detergency-improving action along with the bactericidal or antifungal action. Silver has the bactericidal or antifungal action. For the metal 48, copper or lead cannot be used because of its toxicity. Expensive materials such as gold cannot be adopted in view of costs. The ratio by weight between the tourmaline 46 and the metal 48 is preferably at 10:1 to 1:10.

The cartridge 44 is in the form a hollow cylinder opened at one end thereof and is provided with a multitude of holes 52 at a bottom face 50 thereof. The size of the hole 52 is so determined that in case where the tourmaline mixture 46 and the metal 48 are placed inside the cartridge 44, the tourmaline 46 and the metal 48 do not pass through the holes 52 at the bottom face 50. As shown in Fig. 3, the respective cartridges 44 are set such that the bottom face 50 having a multitude of holes 52 are turned below, and the tourmaline 46 and the metal 48 are placed on the bottom face 50. The respective cartridges 44 are so designed that water is run from the bottom toward the top inside the cartridge 44. More particularly, the respective cartridges 44 are designed in such a way that water passing through the multitude of holes 52 at the bottom face 50 upwardly jets against the tourmaline 46 and the metal 48. The size and number of the holes 52 are so determined that since city water has a high hydraulic pressure, such pressurized water vigorously collides with the tourmaline 46 and the metal 48 in the cartridge 44 thereby causing the tourmaline 46 and the metal 48 to be agitated in the cartridge 44 by the stream of water. For the agitation of the tourmaline 46 and the metal 48 in the cartridge 44 by the passing stream of water, although it may occur to use various means, any hitherto known agitation means may be used. The reason for the agitation of the tourmaline by jetting water against the tourmaline is that the tourmaline and water are frictionally contacted under the agitation, so that the electrodes are dissolved out in water to cleave the clusters of water, thereby generating a large quantity of hydronium ions (H₃O⁺). The jetting of pressurized water, such as city water, through the holes 52 against the tourmaline and the like may omit the provision of an agitation means.

In an instance of practical installment, four cartridges 44, each having an accommodation capacity with an inner diameter of 5 cm and a depth of 7 cm, are put one on another. The tourmaline 46 and the metal 48 are charged in the respective cartridges 44 in such an amount that the tourmaline 46 and the metal 48 can be freely moved within the cartridge 44. The number of the cartridges 44 may be increased or decreased, and only one cartridge 44 having a great capacity may be used. In this way, both the tourmaline 46 and the metal 48 are, respectively, placed in a plurality of cartridges 44, each reduced in capacity. These plural cartridges 44 are connected whereby the agitation efficiency of the tourmaline 46 and the metal 48 can be enhanced by the force of water. The tourmaline 46 accommodated in the cartridges 44 is dissolved in water and disappears in several months. For this, the cartridges 44 are so arranged as to be readily detached such as, for example, by screwing, thereby permitting easy supplement of the tourmaline 46 in the respective cartridges 44. It will be noted that the metal 48, not dissolved in water, need not be supplemented, but the entire cartridge 44 containing the tourmaline 46 and the metal 48 may be replaced by a fresh one. The capacity of the cartridge 44 may vary depending on the flow rate used.
It has been stated that the tourmaline 46 alone or a mixture of the tourmaline 46 and the metal 48 is accommodated in the cartridges 44. The increase of the minus ions in the water passed through the cartridges 44 can be achieved by mutual frictional contact of the particulate tourmaline 46. This is why the tourmaline 46 alone may be accommodated in the cartridges 44. In this connection, however, mixing of the metal 48 with the tourmaline 46 enables the tourmaline 46 to generate a more increased number of minus ions.

The tourmaline 46 has plus and minus electrodes therein. When tourmaline is agitated in water, water (H₂O) is dissociated into a hydrogen ion (H⁺) and a hydroxide ion (OH-).

H₂O → H⁺ + OH- (1)

Further, a hydronium ion (H₃O⁺) having the surface activity is formed from the hydrogen ion (H⁺) and water (H₂O). The amount of the thus formed hydronium ion (H₃O⁺) is far much larger than that generated by means of the ion exchange resin 32.

H₂O + H⁺ → H₃O⁺ (2)

Part of the hydronium ions (H₃O⁺) combines with water (H₂O) to form a hydroxyl ion (H₃O₂⁻) and hydrogen ions (H⁺).

H₃O⁺ + H₂O → H₃O₂ + 2H⁺ (3)

The water passed through the ion exchange resin 32 is subsequently passed through the ion generator 14, whereupon hydronium ions (H₃O⁺), hydroxyl ions (H₃O₂-), H⁺ and OH- are generated in water. It will be noted that the chlorine (Cl) passed through the ion exchange resin 32 and the Na⁺ generated at the ion exchange resin 32 pass through the ion generator 14 as they are without undergoing any reaction.

The water passed through the ion generator 14 is subsequently passed through the rock accommodating container 16 accommodating a rock 54 selected from igneous rocks and containing a large amount of silicon dioxide (i.e. a rock containing about 65 to 76% of silicon dioxide). For the rock 54 selected among igneous rocks (classified into volcanic rocks and plutonic rocks) and containing a large amount of silicon dioxide, mention is made of rhyolites including obsidian, pearlite and pitchstone for volcanic rocks and granites for plutonic rocks. At least one type of rock selected from those mentioned above is accommodated in individual rock accommodating containers 16. The rhyolites such as obsidian, pearlite and pitchstone, or granites bear a minus electron.

The rock (e.g. rhyolites such as obsidian, pearlite and pitchstone, or granites) selected from these igneous rocks and containing a large amount of silicon dioxide has a redox potential of -20 to -240 mV as raw stone. In this connection, the rock 54 used should not be soluble in water and should not be harmful for drinking. The rock accommodating container 16 is in the form of a hollow cylinder having, for example, an inner diameter of 10 cm and a height of 80 cm and accommodates therein the rock 54 selected from igneous rocks and containing a large amount of silicon dioxide in the form of particles with a size, for example, of about 5 mm to 50 mm in such an amount as not to cause a flow rate of water to be lowered.

When the water passed through the ion generator 14 is run through the rock accommodating container 16, e⁻ (minus electron) is added to the water. As a result, the chlorine (Cl) contained in city water is converted to a chlorine ion by means of the minus electron.

Cl + e- → Cl- (4)

This Cl- and the afore-indicated Na⁺ are kept stable in condition as ions. The stable condition means that the conditions of these ions are kept over a long time without evaporation. The afore-indicated hydroxyl ion (H₃O₂-) is also in a stable condition as an ion. When the water is passed through the rock 54, the hydronium ion (H₃O⁺) is increased in number over those in water passed through the ion generator 14, along with a hydroxyl ion (H₃O₂-) and a hydrogen ion (H⁺) being further increased in number.

H₂O + H⁺ → H₃O⁺ (2)

H₃O⁺ + H₂O → H₃O₂- + 2H⁺ (3)

The passage of water through the rock 54 brings about the following reactions other than those indicated above.

OH⁻ + H⁺ → H₂O (5)

2H⁺ + 2e- → 2H₂ (6)

Moreover, when water is passed through the rock accommodating container 16, the redox potential of the water is changed from +340 mV to -20 to -240 mV by means of the minus electrons of the rock 54. Using hot water in place of water, the minus redox potential becomes more stabilized.

As stated hereinabove, water, which is initially passed through the ion exchange resin 32, then through the tourmaline 46 and the metal 48, and finally through the rock 54, (which water is referred to hereinafter as "artificially created water") contains Na⁺, Cl-, H⁺, OH⁻, a hydronium ion (H₃O⁺), and a hydroxyl ion (H₃O₂-). The artificially created water has an electromagnetic wave with a wavelength of 4 to 14 micrometers, the energy of which is at 0.004 watts/cm², and also has a redox potential of -20 to -240 mV.

The results of quality inspection of the artificially created water are shown below. The values of city water are indicated in parentheses for comparison therewith, provided that the same values of city water as those of the artificially created water are indicated as "same".
The nitrous nitrogen and nitric nitrogen: 1.8 mg/l (same), chlorine ion: 6.8 mg/l (9.0 mg/l) general bacteria: 0/ml (same), cyan ion: less than 0.01 mg/l (same), mercury: less than 0.0005 mg/l (same), organic phosphorus: less than 0.1 mg/l (same), copper: less than 0.01 mg (same), iron: less than 0.05 mg/l (less than 0.08 mg/l), manganese: less than 0.01 mg/l (same), zinc: less than 0.005 mg/l (less than 0.054 mg/l), lead: less than 0.01 mg/l (same), hexavalent chromium: less than 0.02 mg/l (same), cadmium: less than 0.005 mg/l (same), arsenic: less than 0.005 mg/l (same), fluorine: less than 0.15 mg/l (same), calcium, magnesium, etc. (hardness): 1.2 mg/l (49.0 mg/l), phenols: less than 0.005 mg/l (same), anionic surface active agent: less than 0.2 mg/l (same), pH value: 6.9 (same), odor : no offensive odor (same), taste: no strange taste (same), chromaticity: 2 degrees (same), and turbidity: 0 degree (one degree).

The artificially created water has the following many features.

### (a) Having surface activity

The artificially created water contains a hydronium ion (H₃O⁺) and a hydroxyl ion (H₃O₂-) and thus, has surface activity (emulsifying activity for O/W type emulsion).

### (b) Having antifungal and antibacterial activities

All of aluminium, a stainless steel and silver used as the metal 48 have the antifungal and antibacterial activities. In case where Na⁺ is generated by means of the ion exchange resin 32, Na⁺ also exhibits the antifungal and antibacterial activities.

### (c) Having a very weak energy (grow light)

Tourmaline emits a very weak energy (an electromagnetic wave having a wavelength of 4 to 14 micrometers). This very weak energy permits large-sized clusters of water to be broken, thereby causing toxic gases and heavy metals included in the clusters to be released to outside, thereby providing water that is good to the human health. The very weak energy is absorbable light that is susceptible to absorption in bodies, animals and plants, and it is believed that the weak energy serves to create such a state as to excite the atoms, molecules and cells, thus giving a good influence on the cells of animals and plants including human beings.

### (d) Having active oxygen scavenging activity

The artificially created water has a redox potential of -20 to -240 mV, and active hydrogen is generated by the action of the minus redox potential, so that the active oxygen inside the human body is reduced by reduction.

As a result of many experiments using the artificially created water and made by the present inventors, it has been found that the artificially created water has the action of suppressing cancer cells occurring in animals. The results of a test of suppressing cancer cells by use of the artificially created water are illustrated.
Initially, cancer cells are planted into a number of rats. The cancer cells used are L-1210 (lab cancer cells), and 20,000 cells were injected into the abdominal cavity of each rat. Eventually, individual rats have ascites cancer. After confirmation of colonization of cancer cells, 18 rats were classified into three groups, each consisting of 6 rats. For a test, ordinary city water is given to the first group as drinking water. The second group is given with artificially created water for drinking water. The third group is given with artificially created water for drinking and also is administered with artificially created water by intravenous injection from a tail. The amount of the artificially created water by injection is at 1.5 cc to 2.0 cc during seven days from commencement of the test, followed by further injection of the same amount as indicated above at intervals of 3 days.

Fundamentally, it departs from common sense to dose 1.5 cc of artificially created water by injection. The dosage of 1.5 cc to a rat by injection calculated for human beings corresponds to a dosage by injection of 4 liters of water. In general, if 1.5 cc of city water is injected into a rat, the rat will be dead within a short time. In this connection, however, the rats that have been injected with 1.5 cc of the artificially created water have never been dead.

The subsequent results revealed that six rats in the first group all were dead at the seventh week. At the time when the seven weeks passed, all the rats of the second and third groups were alive (except for those that fed on each other). Even at the tenth week, all the rats of the second group and the third group were alive. Where cancer cells were planted to rats, it is usual that all rats were dead at the seventh week, for which it is rather strange that all the rats are alive for 10 weeks or over. These results revealed that where the artificially created water is ingested as drinking water or where the artificially created water is not only ingested as drinking water, but also intravenously injected, such water is effective for treating cancer.

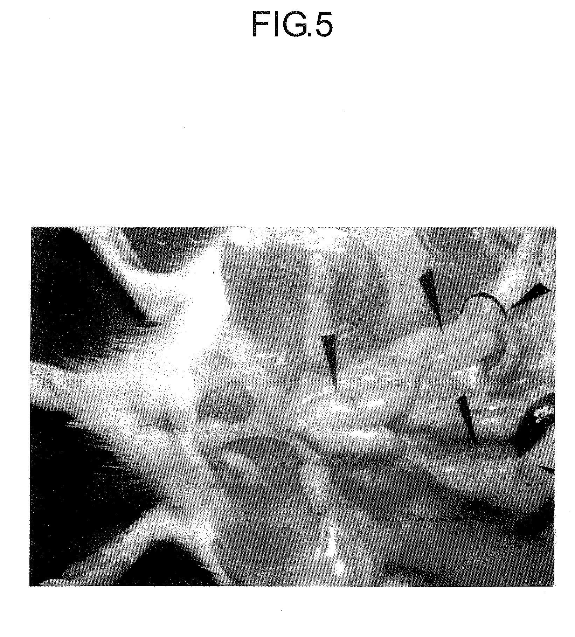

In order to confirm whether the above test (first test) is correct, the same test (second test) was repeated. More particularly, 18 rats whose colonization of cancer cells was confirmed were classified into three groups and tested under the same conditions as set out hereinabove. In this test, however, one of each group was dissected in the course of testing. A rat of each group was dissected at the third to fourth week to confirm the existence of the cancer or how the cancer was in progress. The photographs of the dissected rats of the three groups are shown in Figs. 5 to 8. It will be noted that color photographs corresponding to Figs. 5 to 8 are submitted for reference.

The rat of the first group subjected to dissection at the first day of the fourth week had largely expanded cancer cells, as shown in Figs. 5 and 6. More particularly, it was found that with the rat of the first group, the cancer cells were divided and proliferated. No cancer cell was found at all with respect to the rats of the second group at the fourth day of the fourth week, as shown in Fig. 7. With the rats of the third group at the second day of the third week, no cancer cell existed as shown in Fig. 8. More particularly, it was found that the cancer cells disappeared with respect to the rats of the second and third groups. Thereafter, although all the rats of the first group were dead at the seventh week, the rats of the second and third groups were all alive at the tenth week except for the rats that fed on each other.

As will be apparent from the second test, when the artificially created water is ingested as drinking water or is intravenously injected along with the artificially created water being taken as drinking water, the artificially created water is effective for treating the cancer with respect to the rats. Since remarkable effects are achieved in the animal tests using rats, the water could have a similar effect on men.

It is assumed that the reason why the artificially created water has an effect of suppressing cancer cells from division and proliferation resides in the proton effect (hydrogen ion effect) of the hydrogen ion (H⁺) and the hydronium ion (H₃O⁺) contained in the artificially created water. Cancer cells (microbes) have a cell membrane as a kind of organism. There is a potential difference in H⁺ between the inner and outer sides of the cell membrane, and a substance enters into microbes by utilizing the potential different of H⁺. In this connection, however, the cell membrane has a role of a wall for inhibiting a substance, which must not enter into the microbe, from invasion into the inside. Nevertheless, the artificially created water contains the hydrogen ion (H⁺) and the hydronium ion (H₃O⁺) in large amounts, for which it is considered that such a force as to move the hydrogen ion (H⁺) from the outside toward the inside of the cell membrane against a difference gradient in osmotic pressure of the cell membrane of a microbe is so great (i.e. a proton driving force is great). It is assumed that when the artificially created water containing large amounts of a hydrogen ion (H⁺) and a hydronium ion (H₃O⁺) is infiltrated into microbes, the microbe loses its division and proliferation activities.

The artificially created water is not one obtained by adding some chemical thereto, but is one obtained by using city water or well water as it is. Accordingly, the artificially created water is stable without losing characteristic properties inherent to natural water, and no environmental residue is left. The artificially created water has no influence on the human body. In this way, the artificially created water has safety and stability, like city water and well water, is suited as drinking water, involves no problem in store keeping and the like, and can be handled as ordinary water. Since the artificially created water is ordinary water, there is no concern of side effects of hair loss and the like as would occur by administration of drugs or side effects related to other drugs although it exhibits anticancer action.

### [Example 2]

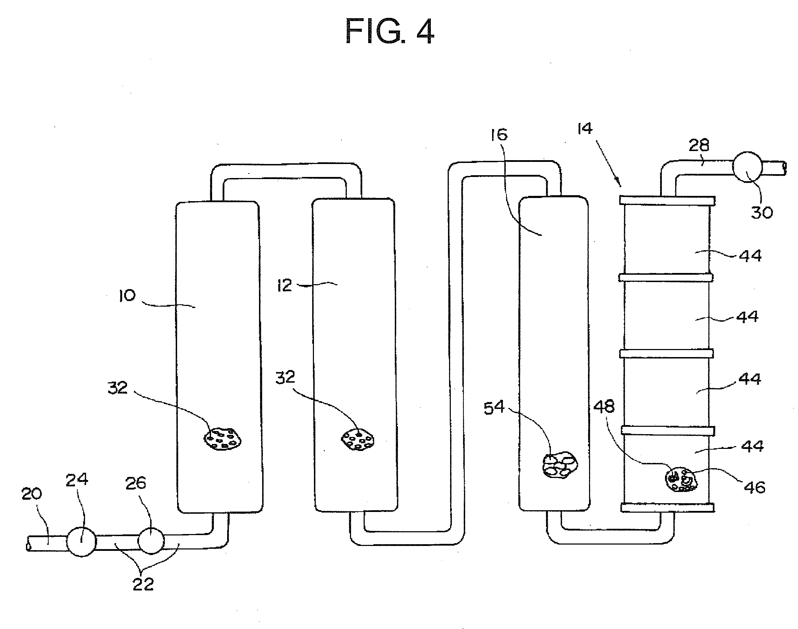

Although, in Example 1, water is passed through the ion exchange resin 32, the tourmaline 46 (or a mixture of the tourmaline 46 and the metal 48), and the rock 54 in this order, water may be successively passed through the ion exchange resin 32, the rock 54, and the tourmaline 46 (or a mixture of the tourmaline 46 and the metal 48). More particularly, as shown in Fig. 4, water is passed through the soft water generator 10, the second soft water generator 12, the rock accommodating container 16 and the ion generator 14 in this order. In this case, the apparatus is so arranged that water moves from lower to upper directions inside the ion generator 14.

In Example 2, water passed through the ion exchange resin 32 subsequently passes through the rock 54. The rock 54 permits e- (a minus electron) to be generated in the water. Eventually, chlorine contained in city water is converted to a chlorine ion by the action of the minus electron.

Cl + e⁻ → Cl- (4)

This Cl- and the Na⁺ generated by means of the ion exchange resin 32 become stabilized as an ion, respectively. It will be noted that water passed through the ion exchange resin 32 may not contain Na⁺ in some case.
As shown in the foregoing formulas (1) and (2), there exist H⁺, OH⁻ and a hydronium ion (H₃O⁺) in the water passed through the ion exchange resin 32. The water passed through the ion exchange resin 32 subsequently undergoes the following reactions by passage through the rock 54.

OH⁻ + H⁺ → H₂O (5)

H₂O + H⁺ → H₃O⁺ (2)

2H⁺ + 2e⁻ → 2H₂ (6)

In these reactions, the hydronium ion (H₃O⁺) generates in amounts larger than that generated by passage through the ion exchange resin 32.
As stated hereinabove, after the passage through the rock 54 subsequent to the ion exchange resin 32, there exist, in water, Na⁺ and OH⁻ that have previously existed in water along with freshly generated Cl- and a hydronium ion (H₃O⁺). The water passed through the rock 54 has a redox potential of -20 to -240 mV. If hot water is used in place of water, the minus redox potential becomes more stabilized.

The water passed through the rock 54 is further passed through the ion generator 14 containing the tourmaline 46 and the metal 48 therein. By the passage, the following reactions take place.

H₂O → H⁺ + OH⁻ (1)

H₂O + H⁺ → H₃O⁺ (2)

The hydronium ion (H₃O⁺) is formed in large amounts. Part of the hydronium ion (H₃O⁺) is converted into a hydroxyl ion (H₃O₂-) as follows.

H₃O⁺ + H₂O → H₃O₂- + 2H⁺ (3)

As a result, the water obtained after passage through the tourmaline 46 and the metal 48 contains previously-existing Na⁺, Cl- and OH- and also a hydronium ion (H₃O⁺) and a hydroxyl ion (H₃O₂-).

More particularly, both the artificially created water obtained in Example 2 and the artificially created water obtained in Example 1 contain Na⁺, Cl- and OH-, a hydronium ion (H₃O⁺), a hydroxyl ion (H₃O₂⁻) and H⁺ and have the same composition. Moreover, they possess an electromagnetic wave of 4 to 14 micrometers having an energy of 0.004 watts/cm² and a redox potential of -20 to -240 mV. As a result, both the artificially created water obtained in Example 2 and the artificially created water obtained in Example 1 have similar effects. Using the artificially created water obtained in Example 2, a test using three groups of rats was conducted. It was found that the results of the test using the artificially created water of Example 2 were similar to those obtained from the artificially created water of Example 1 and thus, the artificially created water of Example 2 could also suppress cancer cells.

### [Example 3]

In Example 3, the first soft water generator 10 and the second soft water generator 12 in Fig. 1 are not used at all, and instead, the ion generator 14 containing the tourmaline 46 (or a mixture of the tourmaline 46 and the metal 48) and the rock accommodating container 16 containing the rock 54 are connected in series. In Example 3, since water is not passed through an ion exchange resin, metal ions such as Ca²⁺, Mg²⁺, Fe²⁺ and the like are not removed from and contained in city water arriving at the ion generator 14. H⁺, OH⁻ or a hydronium ion (H₃O⁺) as shown in the foregoing chemical formulas (1) and (2) is not generated.
In this condition, the city water is passed through the ion generator 14, whereupon a hydrogen ion (H⁺) and a hydroxide ion (OH⁻) are formed.

H₂O → H⁺+ OH⁻ (1)

Of these hydrogen ion (H⁺) and hydroxide ion (OH-), the hydrogen ion (H⁺) combines with water (H₂O) to provide a hydronium ion (H₃O⁺).

H₂O + H⁺ → H₃O⁺ (2)

In this way, H⁺, OH- and a hydronium ion (H₃O⁺) are formed in the water after passage of the ion generator 14 containing the tourmaline 46 and the metal 48 therein. This water has an electromagnetic wave of 4 to 14 micrometers having an energy of 0.004 watts/cm².

When the water passed through the ion generator 14 is further passed through the rock accommodating container 16 containing the rock 54, chlorine present in the city water is initially converted to a chlorine ion by the action of the minus ion.

Cl + e⁻ → Cl- (4)

This Cl- becomes stabilized as an ion. The stabilized state means that an ionic state is kept over a long time without evaporation. A hydronium ion (H₃O⁺) is further generated. Part of the hydronium ion (H₃O⁺) reacts with water to provide a hydroxyl ion (H₃O₂-).

H₃O⁺ + H₂O → H₃O₂- + 2H⁺ (3)

This hydroxyl ion (H₃O₂⁻) also becomes stabilized as an ion.
The passage of the water through the rock 54 leads to the following reactions.

OH- + H⁺ → H₂O (5)

2H⁺ + 2e- → 2H₂ (6)

More particularly, the passage of the water through the rock 54 allows OH⁻, H⁺, a hydronium ion (H₃O⁺) and a hydroxyl ion (H₃O₂-) to exist or be generated, as shown in the foregoing chemical formulas (1), (6), (2) and (3). In addition, after passage of the water through the rock 54, a redox potential of -20 to -240 mV is attained.

In Example 3, water is not passed through an ion exchange resin, so that there is the possibility that metal ions such as such as Ca²⁺, Mg²⁺, Fe²⁺ and the like are contained in the water, which differs from Examples 1 and 2. More particularly, there is some case where water used is hard water, and because no Na⁺ is contained, antifungal and bactericidal activities become slightly poorer.
In this connection, however, the water is passed through the metal 48 and thus, has an electromagnetic wave of a wavelength of 4 to 14 micrometers and a redox potential of -20 to -240 mV. Thus, the resulting water has a very weak energy (grow light) action indicated in (b) before and antifungal and bactericidal activities indicated in (c). Using the artificially created water obtained in Example 3, a test using three groups of rats was conducted. The results of the test using the artificially created water of Example 3 are similar to those of the artificially created water of Example 1, revealing that the artificially created water of Example 3 can suppress cancer cells.

### [Example 4]

Example 4 is such that the ion generator 14 used in Example 3 is substituted with the rock accommodating container 16. More particularly, since water is not passed through the ion exchange resin 32, it is initially passed through the rock 54, followed by passing through the tourmaline 46 (or a mixture of the tourmaline 46 and the metal 48). In this example, metal ions such as Ca²⁺, Mg²⁺, Fe²⁺ and the like are contained to a final stage and no Na⁺ is present in water, like Example 3.
When city water is passed through the rock accommodating container 16, chlorine is converted to a chorine ion by the action of a minus ion.

Cl + e⁻ → Cl⁻ (4)

Next, the water passed through the rock 54 is further passed through a tourmaline mixture 46 and a metal 48 for mixing, whereupon water (H₂O) is dissociated into a hydrogen ion (H⁺) and a hydroxide ion (OH⁻).

H₂O → H⁺ + OH- (1)

Of these hydrogen ion (H⁺) and hydroxide ion (OH-), the hydrogen ion (H⁺) combines with water (H₂O) to provide a hydronium ion (H₃O⁺).

H₂O + H⁺ → H₃O⁺ (2)

More particularly, the passage of water through the rock 54 results in the generation of OH⁻, H⁺ and a hydronium ion (H₃O⁺) as shown in the foregoing chemical formulas (1) and (2). In addition, water passes through the rock 54 has a redox potential of -20 to -240 mV.

The water passed through the rock 54 is subsequently passed through the ion generator 14 containing the tourmaline 46 and the metal 48 therein. Eventually, a hydronium ion (H₃O⁺) is further generated. Part of the hydronium ion (H₃O⁺) is converted to a hydroxyl ion (H₃O₂).

H₃O⁺ + H₂O → H₃O₂⁻ + 2H⁺ (3)

The passage of water through the rock 54 results in the following reaction.

OH⁻ + H⁺ → H₂O (5)

In this way, there are generated, in the water passed firstly through the rock 54 and secondly through the tourmaline mixture 46 and the metal 48 for mixing, Cl⁻, H⁺ and OH⁻, a hydronium ion (H₃O⁺) and a hydroxyl ion (H₃O₂⁻) as is particularly shown in the formulas (4) (1), (2) and (3). In addition, the water has an electromagnetic wave with a wavelength of 4 to 14 micrometers.

In Example 4, since no water is passed through an ion exchange resin, there is a difference from Examples 1 and 2 in that metal ions such as Ca²⁺, Mg²⁺, Fe²⁺ and the like may be contained. If so, water may become hard water in some case, and thus, a detergency effect may lower over the case of Examples 1 and 2. Since no Na⁺ is contained, antifungal and bactericidal activities become slightly lower. In this connection, however, since the water is passed through the metal 48 and has an electromagnetic wave with a wavelength of 4 to 14 micrometers and a redox potential of -20 to -240 mV, thus ensuring the very weak energy (grow light) action of (b) indicated before and antifungal and bactericidal activities of (c), like the Example 3. The artificially created water obtained in Example 4 was used to conduct a test using three groups of rats. The results of the test for the artificially created water of Example 4 are similar to those of the artificially created water of Example 1, revealing that the artificially created water of Example 4 can suppress cancer cells.

It will be noted that in the first and second tests, the case where the artificially created water is taken in the human body as drinking water and the case where the artificially created water is taken in the human body as drinking water along with intravenous injection thereof have been illustrated, no test making use of only intravenous injection of the artificially created water has been conducted. In this connection, however, it is assumed that the intravenous injection of the artificially created water is more effective than the ingestion of the artificially created water in the human body as drinking water because of the direct dosage of the artificially created water into cancer cells. In this sense, a test of intravenous injection of the artificially created water was omitted. In other words, it is considered that the intravenous injection of the artificially created water can more effectively suppress the division and proliferation of cancer cells than the ingestion of the artificially created water in the human body as drinking water.

### [BRIEF DESCRIPTION OF THE DRAWINGS]

[FIG. 1] is a schematic view of an arrangement showing an example of an apparatus for making water having an anticancer activity according to the invention.
[Fig. 2] is a sectional view of a soft water generator used in the making apparatus shown in Fig. 1.
[Fig. 3] is a sectional view of an essential part of an ion generator used in the making apparatus shown in Fig. 1.
[Fig. 4] is a view of an arrangement showing other example of an apparatus for making water having anticancer activity according to the invention.
[Fig. 5] is a dissection photograph of a rat belonging to a first group at the first day of the fourth week.

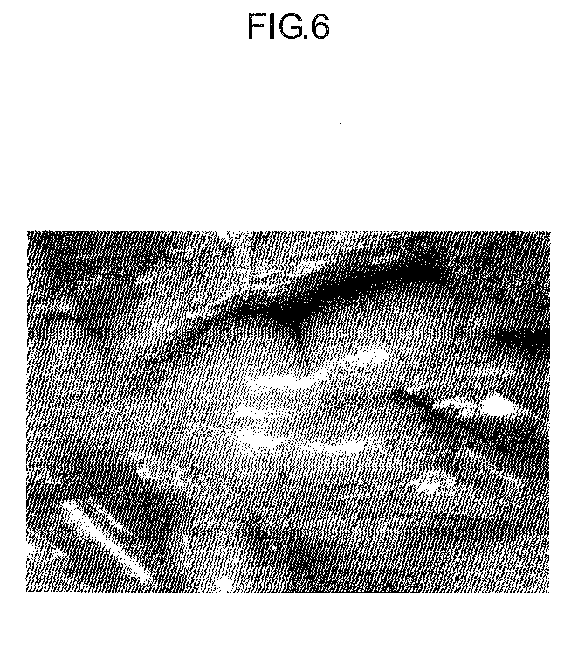

[Fig. 6] is an enlarged photograph of cancer cells in the vicinity of the central portion of Fig. 5.

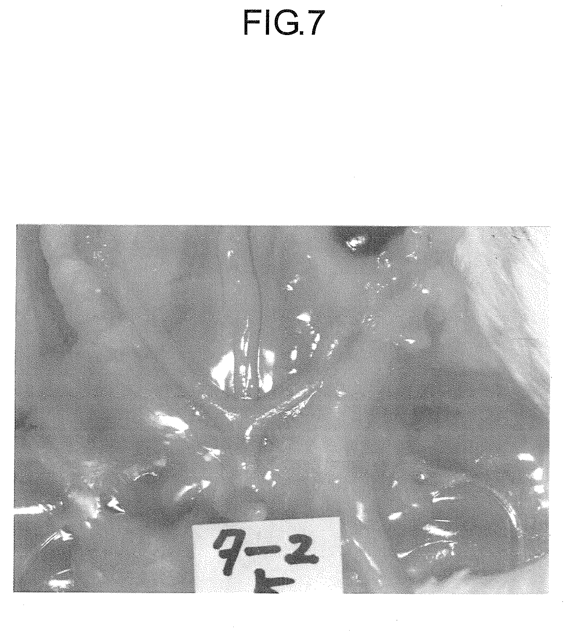

[Fig. 7] is a dissection photograph of a rat belonging to a second group at the fourth day of the fourth week.

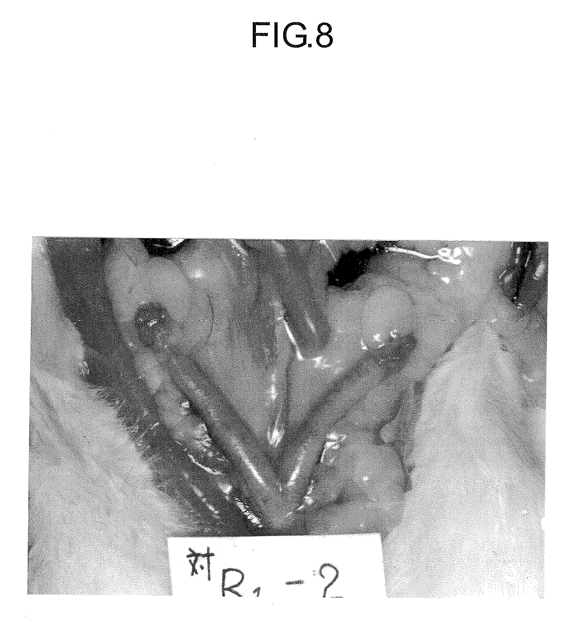

[Fig. 8] is a dissection photograph of a rat belonging to a third group at the second day of the third week.

### [EXPLANATION OF REFERENCE NUMERALS]

- 10: first soft water generator
- 12: second soft water generator
- 14: ion generator
- 16: rock accommodating container
- 32: ion exchange resin
- 46: tourmaline mixture
- 48: metal for mixing
- 54: rock

## Claims

1. A method for making water having anticancer activity, **characterized by** passing water through an ion exchange resin, tourmaline and a rock selected from igneous rocks and containing a large amount of silicon dioxide in this order.

2. A method for making water having anticancer activity according to Claim 1, **characterized in that** a metal that is free of an adverse influence on the human body by dissolution in water is mixed with said tourmaline.

3. A method for making water having anticancer activity according to Claim 1 or 2, **characterized in that** said rock selected from igneous rocks and containing a large amount of silicon dioxide consists of at least one member selected from rhyolites including obsidian, pearlite and pitchstone and granites.

4. A method for making water having anticancer activity according to Claim 3, **characterized in that** said metal consists of at least one member of aluminium, a stainless steel and silver.

5. A method for making water having anticancer activity according to Claim 4, **characterized in that** a ratio by weight between said tourmaline and said metal is at 10:1 to 1:10.

6. A method for making water having anticancer activity according to Claim 5, **characterized in that** said tourmaline is mixed with a ceramic material at a ratio thereof by weight of not less than 10% and heated at 800°C or over.

7. A method for making water having anticancer activity according to Claim 3, **characterized in that** said rock selected from igneous rocks and containing a large amount of silicon dioxide is one obtained by heating at 800°C or over.

8. A method for making water having anticancer activity according to Claims 1 to 3, **characterized in that** said ion exchange resin generates a sodium ion by ion exchange.

9. A method for making water having anticancer activity, **characterized by** passing water through an ion exchange resin, a rock selected from igneous rocks and containing a large amount of silicon dioxide, and tourmaline in this order.

10. A method for making water having anticancer activity according to Claim 9, **characterized in that** a metal that is free of an adverse influence on the human body by dissolution in water is mixed with said tourmaline.

11. A method for making water having anticancer activity according to Claim 9 or 10, **characterized in that** said rock selected from igneous rocks and containing a large amount of silicon dioxide consists of at least one member selected from rhyolites including obsidian, pearlite and pitchstone and granites.

12. A method for making water having anticancer activity according to Claim 11, **characterized in that** said metal consists of at least one member of aluminium, a stainless steel and silver.

13. A method for making water having anticancer activity according to Claim 12, **characterized in that** a ratio by weight between said tourmaline and said metal is at 10:1 to 1:10.

14. A method for making water having anticancer activity according to Claim 13, **characterized in that** said tourmaline is mixed with a ceramic material at a ratio thereof by weight of not less than 10% and heated at 800°C or over.

15. A method for making water having anticancer activity according to Claim 11, **characterized in that** said rock selected from igneous rocks and containing a large amount of silicon dioxide is one obtained by heating at 800°C or over.

16. A method for making water having anticancer activity according to Claims 9 to 11, **characterized in that** said ion exchange resin generates a sodium ion by ion exchange.

17. A method for making water having anticancer activity, **characterized by** passing water firstly through either of tourmaline or a rock selected from igneous rocks and containing a large amount of silicon dioxide, and secondly through the other.

18. A method for making water having anticancer activity according to Claim 17, **characterized in that** a metal that is free of an adverse influence on the human body by dissolution in water is mixed with said tourmaline.

19. A method for making water having anticancer activity according to Claim 18, **characterized in that** said metal consists of at least one member of aluminium, a stainless steel and silver.

20. A method for making water having anticancer activity according to Claim 19, **characterized in that** a ratio by weight between said tourmaline and said metal is at 10:1 to 1:10.

21. A method for making water having anticancer activity according to Claim 20, **characterized in that** said tourmaline is mixed with a ceramic material at a ratio thereof by weight of not less than 10% and heated at 800°C or over.

22. A method for making water having anticancer activity according to Claim 17 or 18, **characterized in that** said rock selected from igneous rocks and containing a large amount of silicon dioxide consists of at least one member selected from rhyolites including obsidian, pearlite and pitchstone and granites.

23. A method for making water having anticancer activity according to Claim 22, **characterized in that** said rock selected from igneous rocks and containing a large amount of silicon dioxide is one obtained by heating at 800°C or over.

24. Water having anticancer activity, **characterized by** passing water through an ion exchange resin, tourmaline and a rock selected from igneous rocks and containing a large amount of silicon dioxide in this order.

25. Water having anticancer activity according to Claim 24, **characterized in that** a metal that is free of an adverse influence on the human body by dissolution in water is mixed with said tourmaline.

26. Water having anticancer activity according to Claim 24 or 25, **characterized in that** said rock selected from igneous rocks and containing a large amount of silicon dioxide consists of at least one member selected from rhyolites including obsidian, pearlite and pitchstone and granites.

27. Water having anticancer activity according to Claim 26, **characterized in that** said metal consists of at least one member of aluminium, stainless steel and silver.

28. Water having anticancer activity according to Claim 27, **characterized in that** a ratio by weight between said tourmaline and said metal is at 10:1 to 1:10.

29. Water having anticancer activity according to Claim 28, **characterized in that** said tourmaline is mixed with a ceramic material at a ratio thereof by weight of not less than 10% and heated at 800°C or over.

30. Water having anticancer activity according to Claim 26, **characterized in that** said rock selected from igneous rocks and containing a large amount of silicon dioxide is one obtained by heating at 800°C or over.

31. Water having anticancer activity according to Claims 24 to 26, **characterized in that** said ion exchange resin generates a sodium ion by ion exchange.

32. Water having anticancer activity, **characterized by** passing water through an ion exchange resin, a rock selected from igneous rocks and containing a large amount of silicon dioxide, and tourmaline in this order.

33. Water having anticancer activity according to Claim 32, **characterized in that** a metal that is free of an adverse influence on the human body by dissolution in water is mixed with said tourmaline.

34. Water having anticancer activity according to Claim 32 or 33, **characterized in that** said rock selected from igneous rocks and containing a large amount of silicon dioxide consists of at least one member selected from rhyolites including obsidian, pearlite and pitchstone and granites.

35. Water having anticancer activity according to Claim 34, **characterized in that** said metal consists of at least one member of aluminium, a stainless steel and silver.

36. Water having anticancer activity according to Claim 35, **characterized in that** a ratio by weight between said tourmaline and said metal is at 10:1 to 1:10.

37. Water having anticancer activity according to Claim 36, **characterized in that** said tourmaline is mixed with a ceramic material at a ratio thereof by weight of not less than 10% and heated at 800°C or over.

38. Water having anticancer activity according to Claim 34, **characterized in that** said rock selected from igneous rocks and containing a large amount of silicon dioxide is one obtained by heating at 800°C or over.

39. Water having anticancer activity according to Claims 32 to 34, **characterized in that** said ion exchange resin generates a sodium ion by ion exchange.

40. Water having anticancer activity, **characterized by** passing water firstly through either of tourmaline or a rock selected from igneous rocks and containing a large amount of silicon dioxide, and secondly through the other.

41. Water having anticancer activity according to Claim 40, **characterized in that** a metal that is free of an adverse influence on the human body by dissolution in water is mixed with said tourmaline.

42. Water having anticancer activity according to Claim 41, **characterized in that** said metal consists of at least one member of aluminium, stainless steel and silver.

43. Water having anticancer activity according to Claim 42, **characterized in that** a ratio by weight between said tourmaline and said metal is at 10:1 to 1:10.

44. Water having anticancer activity according to Claim 43, **characterized in that** said tourmaline is mixed with a ceramic material at a ratio thereof by weight of not less than 10% and heated at 800°C or over.

45. Water having anticancer activity according to Claim 40 or 41, **characterized in that** said rock selected from igneous rocks and containing a large amount of silicon dioxide consists of at least one member selected from rhyolites including obsidian, pearlite and pitchstone and granites.

46. Water having anticancer activity according to Claim 45, **characterized in that** said rock selected from igneous rocks and containing a large amount of silicon dioxide is one obtained by heating at 800°C or over.

## Amended claims

### Amended claims under Art. 19.1 PCT

**1.** Amended) A method for making water having anticancer activity, **characterized by** passing water in the first through an ion exchange resin, next by passing water firstly through either of tourmaline or a rock selected from igneous rocks and containing a large amount of silicon dioxide, and secondly through the other.

**2.** A method for making water having anticancer activity according to Claim 1, **characterized in that** a metal that is free of an adverse influence on the human body by dissolution in water is mixed with said tourmaline.

**3.** A method for making water having anticancer activity according to Claim 1 or 2, **characterized in that** said rock selected from igneous rocks and containing a large amount of silicon dioxide consists of at least one member selected from rhyolites including obsidian, pearlite and pitchstone and granites.

**4.** A method for making water having anticancer activity according to Claim 3, **characterized in that** said metal consists of at least one member of aluminium, a stainless steel and silver.

**5.** A method for making water having anticancer activity according to Claim 4, **characterized in that** a ratio by weight between said tourmaline and said metal is at 10:1 to 1:10.

**6.** A method for making water having anticancer activity according to Claim 5, **characterized in that** said tourmaline is mixed with a ceramic material at a ratio thereof by weight of not less than 10% and heated at 800°C or over.

**7.** A method for making water having anticancer activity according to Claim 3, **characterized in that** said rock selected from igneous rocks and containing a large amount of silicon dioxide is one obtained by heating at 800°C or over.

**8.** A method for mating water having anticancer activity according to Claims 1 to 3, **characterized in that** said ion exchange resin generates a sodium ion by ion exchange.

**9.** Deleted)

**10.** Deleted)

**11.** Deleted)

**12.** Deleted)

**13.** Deleted)

**14.** Deleted)

**15.** Deleted)

**16.** Deleted)

**17.** Deleted)

**18.** Deleted)

**19.** Deleted)

**20.** Deleted)

**21.** Deleted)

**22.** Deleted)

**23.** Deleted)

**24.** Amended) Water having anticancer activity, **characterized by** passing water in the first through an ion exchange resin next by passing water firstly through either of tourmaline or a rock selected from igneous rocks and containing a large amount of silicon dioxide, and secondly through the other.

**25.** Water having anticancer activity according to Claim 24, **characterized in that** a metal that is free of an adverse influence on the human body by dissolution in water is mixed with said tourmaline.

**26.** Water having anticancer activity according to Claim 24 or 25, **characterized in that** said rock selected from igneous rocks and containing a large amount of silicon dioxide consists of at least one member selected from rhyolites including obsidian, pearlite and pitchstone and granites.

**27.** Water having anticancer activity according to Claim 26, **characterized in that** said metal consists of at least one member of aluminium, stainless steel and silver.

**28.** Water having anticancer activity according to Claim 27, **characterized in that** a ratio by weight between said tourmaline and said metal is at 10:1 to 1:10.

**29.** Water having anticancer activity according to Claim 28, **characterized in that** said tourmaline is mixed with a ceramic material at a ratio thereof by weight of not less than 10% and heated at 800°C or over.

**30.** Water having anticancer activity according to Claim 26, **characterized in that** said rock selected from igneous rocks and containing a large amount of silicon dioxide is one obtained by heating at 800°C or over.

**31.** Water having anticancer activity according to Claims 24 to 26, **characterized in that** said ion exchange resin generates a sodium ion by ion exchange.

**32.** Deleted)

**33.** Deleted)

**34.** Deleted)

**35.** Deleted)

**36.** Deleted)

**37.** Deleted)

**38.** Deleted)

**39.** Deleted)

**40.** Deleted)

**41.** Deleted)

**42.** Deleted)

**43.** Deleted)

**44.** Deleted)

**45.** Deleted)

**46.** Deleted)

Statement under Art. 19.1 PCT
Amended claim 1 is combined the original claim 1 and the original claim 9.

Therefore, in a method for making water having anticancer activity, described in the claim 1, it is to include a method for making water having anticancer activity by passing water in order of (1)an ion exchange resin, (2)tourmaline, (3)a rock containing a large amount of silicon dioxide of igneous rock, and a method for making water having anticancer activity by passing water in order of (1)an ion exchange resin, (2)a rock containing a large amount of silicon dioxide of igneous rock, (3)tourmaline.

Amended claim 24 is combined the original claim 24 and the original claim 32.

Therefore, in water having anticancer activity, described in the claim 24, it is to include water having anticancer activity, **characterized by** passing water in order of (1)an ion exchange resin, (2)tourmaline, (3)a rock containing a large amount of silicon dioxide of igneous rock, and water having anticancer activity, **characterized by** passing water in order of (1)an ion exchange resin, (2)a rock containing a large amount of silicon dioxide of igneous rock, (3)tourmaline.

The original claims 2 to 8 and 25 to 31 are not amended.
